# EUROPEAN PATENT SPECIFICATION

(11) **EP 0 926 952 B1**
(45) Date of publication and mention of the grant of the patent: **03.04.2002**
(21) Application number: 97930543.0
(22) Date of filing: 18.07.1997
(51) Int. Cl.: A01N 33/12, A01N 37/02, A01N 25/30, A23K 3/03, A23B 9/26, A23L 3/3517

(54) **AN ANTIMICROBIAL COMPOSITION, A METHOD FOR THE PREPARATION OF SAME**
ANTIMIKROBIELLE ZUSAMMENSETZUNG UND VERFAHREN ZU IHRERE HERSTELLUNG
COMPOSITION ANTIMICROBIENNE ET PROCEDE DE PREPARATION

(30) Priority: 19.07.1996 FI 962908
(43) Date of publication of application: 07.07.1999
(73) Proprietor: KEMIRA CHEMICALS OY, 00101 Helsinki (FI)
(72) Inventor: AHLNÄS, Thomas, FIN-00270 Helsinki (FI)
(74) Representative: Saijonmaa, Olli-Pekka
(86) International application number: FI9700452
(87) International publication number: WO9803066

(56) References cited:
- EP-A- 0 265 202
- EP-A- 0 339 957
- WO-A-88/00795
- WO-A-93/16611
- JP-A- 6 145 003
- SE-B- 318 467
- US-A- 4 585 795
- US-A- 4 828 912

## Description

The invention relates to improved antimicrobial compositions based on organic acids, their preparation, and their use for combatting harmful microbes in the treatment and preservation of organic material and in the disinfection of material surfaces.

An important quality factor of foodstuffs and of feeds of beef cattle and poultry is that they are free of Salmonella, Listeria, E. coli and other such bacteria causing food poisoning.

Salmonella is in general combatted in poultry feeds by using mixtures of organic acids, in particular propionic and formic acids, or mixtures of organic acids and their ammonium salts, or salts of organic acids (Poultry International, July 1994, pp. 40-42). The products are either solutions or solid.

According to US-5 234 703, carcasses, such as chicken carcasses, are disinfected by treating them with a solution which, in addition to acetic, propionic, citric and hydrochloric acid, contains medium-long chain, such as C₁₀, fatty acids. It is reported that the mixture is effective against, for example, Salmonella typhimurium and Escherichia coli.

From publication WO 92/21239 there is known an antimicrobial composition against, for example, Salmonella, this composition contains a polyunsaturated C₅-C₁₄ monocarboxylic acid, such as sorbic acid or a derivative thereof, in combination with a substituted or unsubstituted aromatic carboxylic acid, such as benzoic acid, or a salt or ester thereof. The compositions may also contain organic or inorganic acids.

Surface-active quaternary ammonium compounds are well known bactericidal agents which are known to be effective also against, for example, Salmonella. Thus, according to patent publication US-5 145 853, such an agent, e.g. cetyltrimethylammonium chloride, is used for combatting intestinal diseases, e.g. salmonellosis, in poultry and pigs by feeding the ammonium compound in feed to the animals.

It is known that in general the monobicidal effect of quaternary ammonium compounds weakens in the presence of anionic surface-active agents and polymers, as well as of proteins and blood (K.H. Wallhäusser: Antimicrobial preservatives used by the cosmetic industry, pp. 605-745 in J.J. Kabara (Ed.): Cosmetic and drug preservation, Marcel Dekker, 1984). However, in certain applications, for example, in microbicidal cleansing agents, they have also been formulated together with an anionic surface active detergent or soap, e.g. patent publication US-3 560 390 (Gaines).

Patent publication DD-291914 (Luthardt et al.) discloses fungicidally and bactericidally acting salts of aliphatic or aromatic quaternary ammonium compounds with substituted or unsubstituted salicylate anions, usable in agriculture and horticulture. The compounds include ethanoldiethylammonium salicylate, C₁₂-C₁₈-alkyldimethylammonium salicylate, C₁₂-C₁₈-alkyltrimethylammonium salicylate, C₁₂-C₁₈-alkyldimethylbenzylammonium salicylate, and C₁₆-C₁₈-alkylammonium salicylate. The compositions do not contain free acids.

From publication US-3 578 667 (Wakeman & Coates) there is known the use of salts of quaternary ammonium compounds and certain alicyclic dicarboxylic acids, prepared at a molar ratio of 1:1 and 1:2, as microbicidal preparations against, for example, Staphylococcus and Salmonella.

From patent publication CA-2 082 994 there are known quaternary phosphonium and ammonium salts in the preparation of which a halide counter-ion is replaced by various water-soluble organic ions, and their use as protective agents against harmful microbes in technical applications. The compositions do not contain free acids.

From patent publication EP-127 131 (Hofinger) there is known the preparation of quaternary ammonium compound (R¹R²R³)N⁺(CH₂CH₂O)ₓA- and its use as a disinfectant not corrosive to metal, in which compound R¹ and R² are C₈-C₁₂ alkyl groups, R³ is a C₁-C₄ alkyl group, and the anionic counter-ion A- is made up of acetic acid, propionic acid, glycolic acid, tartronic acid, malic acid, tartaric acid, citric acid, maleic acid, and fumaric acid. There are no free acids present in the compositions.

Patent application EP-537 426 (Makino et al.) also discloses germicidal and fungicidal compounds, which are less corrosive than quaternary ammonium chlorides, for cooling waters of processing machines, air-conditioning systems and other similar applications, in which compounds the halide counter-ion has been replaced by phosphoric acid alkyl ester, phosphoric acid polyoxyethylenealkylether ester, or various carboxylic acids. Compositions which contain free acids are not disclosed.

From patent publication DE-2 344 887 (Eucken & Baginsky) there are known compositions intended for preserving cut flowers fresh, which compositions contain saccharide, 20-64 % by weight, non-oxidizing strong mineral acid 0.04-10 % by weight, sorbic acid 0.01-0.5 % by weight, and quaternary alkyldimethylbenzylammonium chloride 0.01-0.5 % by weight, and possibly succinic acid and/or glyoxylic acid 0.1-5 % by weight and water 35-80 % by weight. The compositions do not contain C₁-C₄-carboxylic acids.

WO-A-9316611 discloses compositions for preserving forage which contain as antimicrobia agent 5-25% benzoic acid or its esters, 60-95% of propionic acid and formic acid, propionic and acetic acid or propionic, formic and acetic acid. The benzoic acid is stabilised by a component (3) such as an organic nitrogen compound, among many other named possible stabilisers, which are said to inhibit crystallisation of the benzoic acid.

There are still a number of deficiencies in agents based on small-molecular carboxylic acids and their mixtures, used in combatting Salmonella and other harmful microbes and in the preservation of material of plant and animal origin. Products in liquid form are spread on the target of use by dispersing the product in nozzles into very fine droplets in order to cause it to be distributed as evenly as possible over the material being treated. However, when the droplet size decreases, an increasing proportion of the product goes to waste along with the air current, in which case the problem of odor is worsened along with the loss. Reduction of the corrosion caused by the products is also desirable. The quantities of acid used could also be reduced by improving the efficacy of the product.

One object of the invention is to reduce the above-mentioned deficiencies.

The invention further includes embodiments the objective of which is, in one application step, to produce a short-term microbicidal acidification effect on the target of disinfection, for example, construction material surfaces, vehicle wheels, protective footwear, and the like, and thereafter to leave on the surfaces a thin oily or waxy protective layer which inhibits microbial growth and protects from corrosion.

The above-mentioned problems have now been solved with a novel antimicrobial composition which is characterized in that it comprises an antimicrobial component which contains as sub-components
a) substituted or unsubstituted C₁-C₄-monocarboxylic acid approximately 50-99.9 % by weight and
b) a microbicidal or microbiostatic cationic organic nitrogen compound approximately 0.1-50 % by weight.

The invention is based on the realization, among other things, that a cationic microbicidal or microbiostatic nitrogen compound is better able to exert its effect in microbes damaged by C₁-C₄-carboxylic acids. Furthermore, at a low pH the plant or animal material to be treated has a smaller quantity of anionic functional groups binding this cationic active agent into an ineffective form than there would be without the presence of C₁-C₄-carboxylic acid; thus the acid also in this manner enhances the antimicrobial properties of cationic agents. The cationic nitrogen compounds according to the invention also change the charge of the droplets in the cationic direction and at the same time improve their adherence to a primarily anionically charged biomaterial, whereby the efficacy of the composition is improved and losses are reduced. By the use of a combination of a cationic active agent and an anionic compound the product is caused to be more effective on the surfaces of the organic material being treated, whereby the antimicrobial action is improved, whereby, for example, the efficacy of a formic-acid-based product in the preservation of a coarse vegetable material, such as whole grain, can be improved and its corrosive action reduced. Furthermore, the cationic active agent or the combination of a cationic active agent and an anionic compound reduces the surface tension of the mixture, thereby facilitating the formation of small droplets in the nozzles and an even distribution of the product.

The invention has thus been made that the efficacy of antimicrobial C₁-C₄-monocarboxylic acid containing preservative compositions against Salmonella and other harmful microbes can be enhanced by adding to them a cationic organic nitrogen compound.

The first subcomponent a) of the antimicrobial component in the antimicrobial composition according to the invention is an unsubstituted or substituted C₁-C₄-monocarboxylic acid, the amount of which is approximately 50-99.9 % by weight, preferably 60-99 % by weight, of the weight of the antimicrobial component. The unsubstituted or substituted C₁-C₄-monocarboxylic acid may be selected, for example, from among the following: formic acid, acetic acid, propionic acid, n-butyric acid, isobutyric acid, glycolic acid, lactic acid, hydroxybutyric acid, pyruvic acid, acetacetic acid, and mixtures thereof. Preferred acids are formic acid, acetic acid, propionic acid, lactic acid, or any mixture thereof, in particular formic acid or a mixture of formic acid and propionic acid.

The second subcomponent b) of the antimicrobial component in the antimicrobial composition according to the invention is a cationic organic nitrogen compound having microbicidal or microbiostatic action. By this is meant in the present invention a compound in the cationically charged part of which the nitrogen is linked to at least one organic chain or ring. The cationic charging of the compound may in the composition according to the invention occur under the effect of the C₁-C₄-monocarboxylic acid present. The amount of this subcomponent is preferably approximately 0.1-10 % by weight, and most preferably approximately 0.5-5 % by weight, of the weight of the antimicrobial component.

In the invention it is possible use, as subcomponent b) inhibiting microbial growth, numerous different cationic organic nitrogen compounds or compounds which form such cations in an acidic environment, either alone or in various combinations. By a Cₙ-Cₘ-alkyl chain is meant hereinafter a straight or branched, saturated or unsaturated, substituted or unsubstituted aliphatic, alicyclic or aromatic alkyl group, each chain length separately or any combination thereof.

Usable ammonium compounds include those the cation of which is of the form R¹-N⁺(R², R³, R⁴), where R¹ is a C₂-C₂₂-alkyl or hydroxyalkyl chain; R² is a C₂-C₂₂-alkyl or hydroxyalkyl chain the same as or different from R¹, or it is a benzyl group or a C₁-C₄-alkylbenzyl group, a C₁-C₄-alkyl or hydroxyalkyl group or a hydrogen atom; R³ and R⁴ are C₁-C₄-alkyl or hydroxyalkyl groups or hydrogen atoms. If the antimicrobial component consists substantially only of a C₁-C₄-monocarboxylic acid and an ammonium compound, R¹ is preferably a C₈-C₂₂-alkyl chain.

The cationic nitrogen compound may also be one which comprises a nitrogen-containing ring, such as a pyridyl ring, where the nitrogen is positively charged in the same manner as in ammonium compounds. As a difference from "normal" ammonium compounds, such compounds can be called heterocyclic ammonium compounds, which are thus within the scope of the invention.

Some examples of nitrogen compounds suitable for subcomponent b) are alkyltrimethylammonium halides, such as cetyltrimethylammonium chloride, myristyltrimethylammonium chloride, coco-alkyltrimethylammonium chloride; dialkyldimethylammonium halides, such as dioctyldimethylammonium chloride, didecyldimethylammonium chloride, dilauryldimethylammonium chloride, di-coco-alkyldimethylammonium chloride, dimyristyldimethylammonium chloride, dicetyldimethylammonium chloride, distearyldimethylammonium chloride, dimethyldi(hydrogenated tallow)ammonium chloride; benzylammonium halides, in particular C₁₂-C₁₆-alkyldimethylbenzylammonium halides, such as dodecyldimethylbenzylammonium chloride or dodecylethylbenzyldimethylammonium chloride; tertiary amines, such as decyldimethylamine, lauryldimethylamine, myristyldimethylamine, cetyldimethylamine, palmityldimethylamine; secondary amines, such as dioctylamine, didodecylamine, etc.; primary amines, such as octylamine, decylamine, dodecylamine, oleylamine, alkyldiaminoethylglycine hydrochloride; cyclic ammonium compounds, such as cyclohexylamine; or salts of alkyl derivatives of pyridine, imidazole, pyrrole, isoquinoline or morpholine, some examples being cetylpyridinium chloride and laurylisoquinolinium chloride; polycationic ammonium compounds, such as polyhexamethylenebiguanidine hydrochloride. In the said ammonium compounds, the chloride may also be replaced by some other anion, for example, bromide, formiate or acetate, the latter of which may be formed as subcomponent a) and amine are mixed with each other. In addition to the compounds listed, compounds suitable for use also include compounds where the lengths of the alkyl chains deviate from those mentioned, or where, in the place of one or more methyl groups, there is, for example, an ethyl group or a hydroxyethyl group. The alkyl chains of the cationic compounds may also contain heteroatoms, such as ester or amide groups, or double bonds which promote the biodegradability of the compound. Nitrogen compounds, either alone or in various combinations, are used as subcomponent b) of the formulations.

In particular when subcomponent c) is used in the composition according to the invention in the manner described in the following passage, subcomponent b) may preferably also be a relatively small-molecular amino compound, where group R¹ is preferably a C₂-C₁₈-alkyl or -hydroxyalkyl. Some examples are triethylamine, cyclohexylamine, dimethylamino-2-methyl-1-propanol, diethylaminoethanol, 2-amino-2-methyl-1-propanol, aminobutanol, monoethanolamine, dimethylaminoethanol, diethanolamine, 2-amino-2-ethyl-1,3-propanediol, morpholine, triethanolamine, piperazine, aminoethylethanolamine, ethylenediamine, N,N,N',N'-tetracis-(2-hydroxypropyl)-ethylenediamine, and many others of the type.

According to one embodiment of the invention, additionally subcomponent c), which is an organic acid containing a C₅-C₂₂-alkyl or -aryl group, preferably carboxylic acid, is used in the composition according to the invention in an amount of approximately 0.1-10 % by weight, preferably approximately 0.5-5 % by weight.

As subcomponent c) it is possible to use numerous different organic acids, either alone or in combinations. Such acids include unsubstituted or substituted mono- and polycarboxylic acids and hydroxyacids having an approximately C₅-C₂₂-hydrocarbon chain, such as caproic acid, caprylic acid, capric acid, lauric acid, myristinic acid, myristoleic acid, palmitic acid, palmitoleic acid, stearic acid, oleic acid, elaidic acid, linolic acid, linoleic acid, linoelaidic acid, arachidonic acid, abietic acid, sorbic acid, ascorbic acid, cinnamic acid, tartaric acid, malic acid, benzoic acid, p-hydroxybenzoic acid, and salicylic acid. Especially usable are acids which themselves have properties inhibiting microbial growth, such as benzoic acid, parahydroxybenzoic acid, salicylic acid, sorbic acid, caprylic acid, capric acid, lauric acid, myristic acid, myristoleic acid, palmitic acid, palmitoleic acid, linolic acid, linoleic acid, or arachidonic acid. Acids also usable include aminocarboxylic acids and alkyaminocarboxylic acids, for example, dodecylaminopropionic acid. Also usable are polymeric polycarboxylic acids, such as polyacrylic acid, or copolymeric acids formed by acrylic acids and unsaturated compounds polymerizable therewith. Alongside carboxylic acids, also usable are other unsubstituted or substituted organic acids the anions of which are, for example, sulfonates, sulfates or phosphates.

In connection with the compounds listed, by a Cₙ-Cₘ alkyl chain is meant a straight or branched, saturated or unsaturated, substituted or unsubstituted, aliphatic, alicyclic or aromatic alkyl group, each chain length separately or any combination thereof.

The compounds of subcomponent c) may be added to the compositions in the form of either acids or salts, for example, in the form of alkali metal salts or ammonium salts, or esters.

It is preferable to select subcomponent c) from among compounds which themselves have microbicidal or microbiostatic properties.

According to one particularly preferred embodiment of the invention, the composition comprises an antimicrobial component which contains
a) an unsubstituted or substituted C₁-C₄-monocarboxylic acid approximately 50-99.9 % by weight,
b) a microbicidal cationic organic nitrogen compound approximately 0.1-10 % by weight.
c) an organic acid containing a C₅-C₂₂ alkyl or aryl group, approximately 0.1-10 % by weight and
d) water approximately 0-30 % by weight.

The compositions according to the invention may be prepared simply by mixing together subcomponents a) and b).

According to one preferred embodiment, subcomponent b) and subcomponent c) are separately caused to react with each other so that there forms between the cation of subcomponent b) and the anion of subcomponent c) an addition compound, an anion-cation complex, an "ancat" or "catan" compound or salt, which is here called an ancat compound, and which compound is then mixed with subcomponent a).

The ancat compound may be prepared, for example, by mixing subcomponents b) and c) together at the desired molar ratio, either without a medium or by using a suitable solvent, for example, water, as a medium. The preparation can be conducted by, for example, the following methods 1, 2 and 3:
- In method 1, subcomponent b) and subcomponent c) are mixed together at the desired molar ratio, being heated cautiously when necessary. This method is suitable especially when subcomponent b) is a primary amine and subcomponent c) is an organic acid.
- Method 2 is particularly suited for the preparation of mutual salts of water-soluble cationic and water-soluble anionic compounds. The anionic component c) and the cationic component b) are separately dissolved in water, whereafter the solutions are combined. In a mixture close to a molar ratio of 1:1, the ancat compound separates from the aqueous phase as an oily or paste-like sediment or as a waxy sediment, depending on the temperature and the raw materials, and can be separated from the aqueous phase by any known method. When necessary, the initial components are removed from the product by a water wash. In connection with the method it is also possible to use an auxiliary solvent which is poorly soluble in water, in which case the organic phase with the ancat compound is separated from the aqueous phase and the organic solvent is removed, when necessary, by evaporation before the product is combined with subcomponent a).
- Method 3. The anionic component c) and the cationic component b) are separately dissolved in a similar or different organic solvent and the solutions are combined, or they are dissolved successively or simultaneously in the same solvent or solvent mixture and finally the solvents are removed by evaporation.

Method 2 produces a maximally halogen-free product; the oily or waxy ancat layer forming from it on metal surfaces after the evaporation of the acid component a) has advantageous properties in terms of corrosion.

The amine of subcomponent b) may in certain cases react with the acid of subcomponent c) so that also the corresponding amide is formed in the ancat compound.

The ancat compounds or the subcomponents thereof are in a dissolved, emulsified or dispersed state in a continuous phase consisting of subcomponent a). It is to be noted that, when the ancat compound is mixed with subcomponent a), which contains acids stronger than subcomponent c), the ancat compound breaks down so that subcomponent c) is present primarily in an acid form in the product according to the invention. When subcomponent a) becomes absorbed into the target of use or evaporates, the ancat compound can re-form on the surfaces treated.

By means of the ancat compound it is possible to improve the solubility of poorly soluble subcomponents, such as benzoic acid or salicylic acid, in compositions according to the invention and to inhibit their precipitation.

Preferred ancat compounds for compositions according to the invention include alkylpyridiniumbenzoate, -parahydroxybenzoate or -salicylate, alkylbenzyldimethylammoniumbenzoate, -parahydroxybenzoate or -salicylate, alkyltrimethylammoniumbenzoate, -parahydroxybenzoate or -salicylate, or primary alkylammoniumbenzoate, -parahydroxybenzoate or -salicylate.

At conventional temperatures the preservatives according to the invention are either in a solution form or solid. The solid state has the advantages of, for example, better safety in transport, lesser odor and ease of dispensing. According to one preferred embodiment of the invention, the composition comprises, in addition to the antimicrobial component also a solid, particulate carrier. In such a case it is preferable if the mutual weight ratio of the antimicrobial component to the particulate carrier is within a range of 1:10-10:1, preferably within a range of 3:7-7:3. Since what is concerned is the impregnation of a particulate carrier with a liquid acid solution, the specific surface of the particulate carrier must be large, or it must be otherwise absorbent. The average particle size of the particulate carrier is preferably approximately 0.01-20 mm.

The said particulate substance is preferably one or several of the following: inorganic particulate substances based on oxides of silicon and/or aluminum, such as siliceous earth, moler, silicic acid, silica, alumina, bentonite, montmorillonite, perlite, steatite, chlorite, expanded vermiculite, calcium silicate, sodium aluminum silicate, zeolites, inorganic salts poorly soluble in water and organic acids, and organic substances such as synthetic polymers, starch, cellulose, cellulose derivatives, lignin, lignin derivatives, and solid feed raw materials.

Solid compositions are prepared by impregnating an inorganic or organic absorbent material, such as silica, clay minerals and cellulose, or mixtures thereof, with a solution-form composition as such Solid compositions may also be prepared by impregnating similar or different absorbent materials with liquid ingredients of the composition or liquid combinations of ingredients, the absorbent materials being finally mixed with each other. At the same time it is possible to add solid sub-components b) and c), if any, of the antimicrobial component, and possibly other solid ingredients, such as animal nutrients and other feed components.

Finally the invention relates to the use of any composition described above as a substance inhibiting the growth of harmful microbes in feed raw materials and preparations and the like. What is concerned in this case may be the adding of the composition as a treatment substance or an additive to a feed raw material or to animal feed. The composition may also be used for ensiling or the preservation grain, or for the disinfection of packaging materials, apparatus and equipment, vehicles and other technical equipment and materials, footwear and other protective means, as well as animal sheds, silos and other premises.

The compositions according to the invention may, in addition to the ingredients mentioned above, also contain other compounds enhancing preservation efficacy, for example, an ester formed by a substituted or unsubstituted benzoic acid, such as benzoic acid or hydroxybenzoic acid, with a C₁-C₄ alcohol, for example, methyl, ethyl, propyl or butyl benzoate, parahydroxybenzoate or salicylate; monoglycerides of C₈-C₁₆ carboxylic acids; or other bactericidal or bacteriostatic surfactant compounds; salts, e.g. salts of magnesium, zinc and copper; animal nutrients; or compounds controlling the salt content of the mixture or buffering its pH, e.g. ammonium phosphate or potassium sulfate; imidazolidinyl urea, phenolic compounds and sulfonic acids, e.g. lignosulfonates; or aldehydes, e.g. formaldehyde. Furthermore, it is possible to add to the mixture components which serve as antioxidants, e.g. butylated hydroxyanisol or hydroxytoluene or esters of gallus acid. They may further contain complexing agents which are known, for example on the basis of publication J.R. Hart: Chelating agents as preservative potentiators; pp. 323-337 in J.J. Kabara (ed.): Cosmetic and drug preservation, Marcel Dekker 1984, to sensitize microbes to the effect of quaternary ammonium compounds. Some examples of suitable complexing agents are nitriloacetic acid, ethylenediaminetetraacetic acid, hydroxyethylethylenediaminetriacetic acid, diethylenetriaminepentaacetic acid and cyclohexanediaminetetraacetic acid.

When necessary, it is also possible to use in the compositions other additives, for example, for improving their transport, dissolving or wetting properties, such as alcohols, aminoalcohols, sugars, salts and surfactants.

In addition to subcomponent a) of the antimicrobial component, the compositions according to the invention may also contain other organic or inorganic compounds which react acidically with water, such as acids or polyacids, or compounds which form acids in solution. Suitable inorganic acids include phosphoric acid, sulfuric acid, hydrochloric acid, and nitric acid. The acids may also be in a partly neutralized form. Acid-forming compounds include acidically reacting salts and acid anhydrides.

Compositions according to the invention can thus be used for the treatment of material of plant or animal origin intended for animal feed, the objective being to kill bacteria passing into the material or possibly already present therein, or to inhibit their proliferation to the extent that diseases caused by the microbes will not be caused in animals or in persons coming into contact with the feeds or animals. Feeds containing the largest concentrations of certain compositions according to the invention may also serve to heal intestinal infections in animals.

For the treatment of feed and of feed raw materials, subcomponent b) and subcomponent c) possibly used are, of course, selected from among compounds which are as safe as possible for domestic animals and humans, and which are primarily among the chemicals approved for the given applications in the legislation and directives.

When a composition according to the invention is used. it is sprayed or sprinkled, for example, into the feed raw material. Thus the small-molecular subcomponent a) becomes impregnated relatively taster into the material being treated, possibly becomes at least partly neutralized under the action of its basic or pH-buffering ingredients, or evaporates. Subcomponents b) and c) remain in relatively larger quantities on the surfaces of the material treated, and also on the surfaces of the microbes being combatted. On the latter the compositions according to the invention tend to form an oily or waxy ancat salt film. In this form their ability to become adsorbed to surfaces is greater than that of compositions without subcomponent c).

Compositions according to the invention are also suitable for disinfecting treatment of various materials, equipment and surfaces, for example, in cowsheds and other animal sheds.

The compositions can also be used in other applications of C₁-C₄ carboxylic acids.

The compositions are suitable for the combatting or inhibition of highly various microbes, some examples being gram-negative and gram-positive bacteria, e.g. Salmonella, Escherichia coli, Pseudomonas, Clostridium, Listeria, Campylobacter and Yersinia, or molds, yeasts and fungi, e.g. Fusarium, Aspergillus, Penicillium, Saccharomyces and Candida.

Below, a few embodiment examples and reference examples are given, their only purpose being to elucidate the invention. All the percentages are percentages by weight.

### Example. Evaporation test

The following compositions were prepared (amounts in wt. %):

| **Ingredient** | **A** | **B** | **C** | **D** | **E** | **F** | **G** |
|---|---|---|---|---|---|---|---|
| | ref. | | | | | | |
| Formic acid | 56 | 54.9 | 53.8 | 51.5 | 54.9 | 53.8 | 51.5 |
| Propionic acid | 29 | 28.4 | 27.8 | 26.7 | 28.4 | 27.8 | 26.7 |
| Benzoic acid | 9 | 8.8 | 8.6 | 8.3 | 8.8 | 8.6 | 8.3 |
| Octylamine | - | 2 | 4 | 8 | - | - | - |
| Ethanolamine | - | - | - | - | 2 | 4 | 8 |
| Water | 6 | 5.9 | 5.8 | 5.5 | 5.9 | 5.8 | 5.5 |
| Molar ratio (BA = benzoic acid) | | | | | | | |
| octylamine:BA | - | 0.21 | 0.44 | 0.91 | - | - | - |
| ethanolamine:BA | - | - | - | - | 0.41 | 0.84 | 1.74 |

The mixtures remained stable at room temperature.

When the solutions were dried on Petri dishes, solid benzoic acid crystallized out from sample A, in samples B-D the residue was more or less oily, and in samples EG it was waxy. A corresponding mixture was also made with cyclohexylamine in accordance with the invention, and an oily residue was obtained from it.

### PREPARATION EXAMPLES

### Examples 1-6

The following compositions were prepared by dissolving first benzoic acid and then octylamine in the mixture containing the other ingredients (amounts in % by weight).

| **Ingredient Example** | **1** | **2** | **3** | **4** | **5** | **6** |
|---|---|---|---|---|---|---|
| Formic acid | 76.0 | 74.4 | 71.2 | 73.6 | 72.0 | 68.8 |
| Phosphoric acid | 1.9 | 1.9 | 1.8 | 1.8 | 1.8 | 1.7 |
| Water | 17.1 | 16.7 | 16.0 | 16.6 | 16.2 | 15.5 |
| Benzoic acid | 3.0 | 3.0 | 3.0 | 6.0 | 6.0 | 6.0 |
| Octylamine | 2.0 | 4.0 | 8.0 | 2.0 | 4.0 | 8.0 |
| Molar ratio octylamine benzoic acid | | | | | | |
| | 0.63 | 1.26 | 2.52 | 0.32 | 0.63 | 1.26 |

In the compositions of Examples 1-6, the benzoic acid remained dissolved at room temperature, whereas without amine, benzoic acid precipitates already within an hour.

### VARIOUS Ancat SALTS

### Example 7 Benzoic acid - octylamine.

0.4 g of benzoic acid was dissolved in 0.4 g of n-octylamine while the mixture was being heated (molar ratio octylamine:benzoic acid 1.06). A sticky, oily compound was obtained. When it was used for the preparation of formic acid based formulations for feed preservation, it dissolved when the mixture was stirred vigorously.

### Examples 8-24 Lowest inhibiting concentrations (Bioscreen tests)

For the tests, dilution series of microbial cultures were prepared in peptone water (pH 6) by using various amounts of the preservatives of Table 1. Bacterial growth was monitored for 48 h by using a Bioscreen apparatus (Labsystems, Finland). The apparatus automatically measures from a sample series turbidity which indicates bacterial growth as a function of time. Reference mixtures 1-3 and example compositions 8-24 according to the invention were investigated.

**Table 1.**

| | |
|---|---|
| **Reference 1** | 80 % formic acid + 2 % H₃PO₄ + 18 % water |
| **Reference 2** | 95 % reference mixture 1 + 2.5 % ethylbenzoatc + 2.5 % sodium benzoate |
| **Reference 3** | 90 % reference mixture 1 + 5 % ethylbenzoate + 5 % sodium benzoate |
| **Example 8** | 95 % reference mixture 1 + 2.5 % ethylbenzoate + 2.5 % Kloramin-T/benzalkonium Cl⁻ |
| **Example 9** | 92.5 % reference mixture 1 + 5 % ethylbenzoate + 2.5 % Kloramin-T/benzalkonium Cl⁻ |
| **Example 10** | 90 % reference mixture 1 + 5 % ethylbenzoate + 5 % Kloramin-T/benzalkonium Cl⁻ |
| **Example 11** | 95 % reference mixture 1 + 2.5 % ethylbenzoate + 2.5 % Na benzoate/cetylpyridinium Cl- |
| **Example 12** | 92.5 % reference mixture 1 + 5 % ethylbenzoate + 2.5 % Na benzoate/cetylpyridinium Cl- |
| **Example 13** | 90 % reference mixture 1 + 5 % ethylbenzoate + 5 % Na benzoate/cetylpyridinium Cl⁻ |
| **Example 14** | 95 % reference mixture 1 + 2.5 % Ampholac YJH-40 + 2.5 % Na benzoate/cetylpyridinium Cl- |
| **Example 15** | 92.5 % reference mixture 1 + 5 % Ampholac YJH-40 + 2.5 % Na benzoate/cetylpyridinium Cl- |
| **Example 16** | 90 % reference mixture 1 + 5 % Ampholac YJH-40 + 5 % Na benzoate/cetylpyridinium Cl- |
| **Example 17** | 95% reference mixture 1 + 2.5% Amphoteen 24 + 2.5% Na benzoate/cetylpyridinium Cl- |
| **Example 18** | 92.5 % reference mixture 1 + 5 % Amphoteen 24 + 2.5 % Na benzoate/cetylpyridinium Cl- |
| **Example 19** | 90 % reference mixture 1 + 5 % Amphoteen 24 + 5 % Na benzoate/cetylpyridinium Cl⁻ |
| **Example 20** | 95% reference mixture 1 + 2.5% ethylbenzoate + 2.5% Na benzoate/cetylpyridinium Cl⁻ |
| **Example 21** | 92.5 % reference mixture 1 + 5 % ethylbenzoate + 2.5 % Na benzoate/cetylpyridinium Cl⁻ |
| **Example 22** | 90 % reference mixture 1 + 5 % ethylbenzoate + 5 % Na benzoate/cetylpyridinium Cl⁻ |
| **Example 23** | 95 % reference mixture 1 + 5 % Ampholac YJH-40 |
| **Example 24** | 95 % reference mixture 1 + 5 % Amphoteen 24 |

Ampholac YJH-40 is octyldiaminodipropionate and Amphoteen 24 is C₁₂-C₁₄-alkyldimethylbetaine.

For the Bioscreen test, sample series which contained a microbe inoculum were prepared, and microbicides were dosed into them in amounts of 0.02, 0.04, 0.06, 0.08 and 0.1 %. Efficacy against the micro-organisms in Table 2 was tested.

**Table 2.**

| Microbes used in the investigation | |
|---|---|
| **M1** | Salmonella infantis |
| **M2** | Salmonella agona |
| **M3** | Salmonella montevideo |
| **M4** | Salmonella schwartzgrund |
| **M5** | Salmonella seftenberg |
| **M6** | Salmonella 4,12:b |
| **M7** | Escherichia coli |
| **M8** | Listeria monocytogenes |

The smallest inhibiting concentrations were those shown in Table 3.

**Table 3.**

| Results with samples of Examples 8-24 | | | | | | | | |
|---|---|---|---|---|---|---|---|---|
| **MICROBE: SAMPLE** | **M1** | **M2** | **M3** | **M4** | **M5** | **M6** | **M7** | **M8** |
| **Ref. 1** | >0.1 | >0.1 | >0.1 | >0.1 | >0.1 | >0.1 | >0.1 | >0.1 |
| **Ref.2** | >0.1 | >0.1 | >0.1 | >0.1 | >0.1 | >0.1 | >0.1 | >0.1 |
| **Ref.3** | >0.1 | >0.1 | >0.1 | >0.1 | >0.1 | >0.1 | >0.1 | >0.1 |
| **Ex.8** | 0.06 | 0.06 | 0.06 | 0.06 | 0.06 | 0.06 | 0.04 | 0.02 |
| **Ex.9** | 0.06 | 0.06 | 0.06 | 0.06 | 0.06 | 0.06 | 0.04 | 0.02 |
| **Ex. 10** | 0.04 | 0.04 | 0.04 | 0.04 | 0.04 | 0.04 | 0.02 | 0.02 |
| **Ex. 11** | 0.04 | 0.04 | 0.04 | 0.04 | 0.04 | 0.04 | 0.02 | 0.02 |
| **Ex. 12** | 0.04 | 0.04 | 0.04 | 0.04 | 0.04 | 0.04 | 0.02 | 0.02 |
| **Ex. 13** | 0.02 | 0.02 | 0.02 | 0.02 | 0.02 | 0.02 | 0.02 | 0.02 |
| **Ex. 14** | 0.04 | 0.04 | 0.04 | 0.04 | 0.04 | 0.04 | 0.02 | 0.02 |
| **Ex. 15** | 0.04 | 0.04 | 0.04 | 0.04 | 0.04 | 0.04 | 0.02 | 0.02 |
| **Ex. 16** | 0.02 | 0.02 | 0.02 | 0.02 | 0.02 | 0.02 | 0.02 | 0.02 |
| **Ex. 17** | 0.04 | 0.04 | 0 04 | 0.04 | 0.04 | 0.04 | 0.02 | 0.02 |
| **Ex. 18** | 0.04 | 0.04 | 0.04 | 0.04 | 0.04 | 0.04 | 0.02 | 0.02 |
| **Ex. 19** | 0.02 | 0.02 | 0.02 | 0.02 | 0.02 | 0.02 | 0.02 | 0.02 |
| **Ex. 20** | 0.04 | 0.04 | 0.04 | 0.04 | 0.04 | 0.04 | 0.02 | 0.02 |
| **Ex. 21** | 0.04 | 0.04 | 0 04 | 0.04 | 0.04 | 0.04 | 0.02 | 0.02 |
| **Ex. 22** | 0.02 | 0.02 | 0.02 | 0.02 | 0.02 | 0.02 | 0.02 | 0.02 |
| **Ex. 23** | >0.1 | >0.1 | >0.1 | >0.1 | >0.1 | >0.1 | >0.1 | >0.1 |
| **Ex. 24** | >0.1 | >0.1 | >0.1 | >0.1 | >0.1 | >0.1 | >0.1 | >0.1 |

The results show that a cationic microbicidal compound works also in the presence of an anionic compound, and the efficacy is excellent as compared with the known reference samples containing formic acid and benzoates.

### Examples 25-48 Lowest inhibiting concentrations 2

The lowest inhibiting concentrations of the following liquid formulations against various micro-organisms were tested, the ancat combinations used in the formulations were made at a molar ratio of 1:1:

| | |
|---|---|
| **Example 25** | 97.5 % formic acid + 2.5 % (octanoic acid + cetylpyridine chloride) |
| **Example 26** | 95 % formic acid + 5 % (octanoic acid + cetylpyridine chloride) |
| **Example 27** | 97.5 % lactic acid + 2.5 % (octanoic acid + cetylpyridine chloride) |
| **Example 28** | 95 % lactic acid + 5 % (octanoic acid + cetylpyridine chloride) |
| **Example 29** | 97.5 % acetic acid + 2.5 % (octanoic acid + cetylpyridine chloride) |
| **Example 30** | 95 % acetic acid + 5 % (octanoic acid + cetylpyridine chloride) |
| **Example 31** | 97.5 % propionic acid + 2.5 % (octanoic acid + cetylpyridine chloride) |
| **Example 32** | 95 % propionic acid + 5 % (octanoic acid + cetylpyridine chloride) |
| **Example 33** | 97.5 % formic acid + 2.5 % (adipic acid + benzalkonium chloride) |
| **Example 34** | 95 % formic acid + 5 % (adipic acid + benzalkonium chloride) |
| **Example 35** | 97.5 % lactic acid + 2.5 % (adipic acid + benzalkonium chloride) |
| **Example 36** | 95 % lactic acid + 5 % (adipic acid + benzalkonium chloride) |
| **Example 37** | 97.5 % acetic acid + 2.5 % (adipic acid + benzalkonium chloride) |
| **Example 38** | 95 % acetic acid + 5 % (adipic acid + benzalkonium chloride) |
| **Example 39** | 97.5 % propionic acid + 2.5 % (adipic acid + benzalkonium chloride) |
| **Example 40** | 95 % propionic acid + 5 % (adipic acid + benzalkonium chloride) |
| **Example 41** | 97.5 % formic acid + 2.5 % (octanoic acid + benzalkonium chloride) |
| **Example 42** | 95 % formic acid + 5 % (octanoic acid + benzalkonium chloride) |
| **Example 43** | 97.5 % lactic acid + 2.5 % (octanoic acid + benzalkonium chloride) |
| **Example 44** | 95 % lactic acid + 5 % (octanoic acid + benzalkonium chloride) |
| **Example 45** | 97.5 % acetic acid + 2.5 % (octanoic acid + benzalkonium chloride) |
| **Example 46** | 95 % acetic acid + 5 % (octanoic acid + benzalkonium chloride) |
| **Example 47** | 97.5 % propionic acid + 2.5 % (octanoic acid + benzalkonium chloride) |
| **Example 48** | 95 % propionic acid + 5 % (octanoic acid + benzalkonium chloride) |

In the above, "benzalkonium chloride" is a product name for a composition which was a mixture of alkylbenzyldimethylammonium chlorides, primarily C₁₂-C₁₆-alkyldimethylbenzylammonium chloride. (Reference: CTFA Cosmetic Ingredients Dictionary, 3rd edition. The Cosmetic, Toiletry and Fragrance Association, USA 1982.)

For the Bioscreen test, series containing a microbe inoculum and 0.02, 0.04, 0.06, 0.08 and 0.1 % microbicides were prepared. The microbes investigated were:
**M1** Salmonella infantis
**M2** Saccharomyces cerevisiae
**M3** Candida lambica
**M4** Listeria monocytogenes
**M5** Escherichia coli

The lowest inhibiting concentrations in the test solutions were:

| **MICROBE SAMPLE** | **M1** | **M2** | **M3** | **M4** | **M5** |
|---|---|---|---|---|---|
| **Example 25** | 0.06 | 0.06 | 0.02 | 0.02 | 0.02 |
| **Example 26** | 0.02 | 0.02 | 0.02 | 0.02 | 0.02 |
| **Example 27** | 0.06 | 0.06 | 0.02 | 0.02 | 0.02 |
| **Example 28** | 0.02 | 0.02 | 0.02 | 0.02 | 0.02 |
| **Example 29** | 0.06 | 0.08 | 0.02 | 0.02 | 0.02 |
| **Example 30** | 0.02 | 0.04 | 0.02 | 0.02 | 0.02 |
| **Example 31** | 0.06 | 0.06 | 0.02 | 0.02 | 0.04 |
| **Example 32** | 0.02 | 0.04 | 0.02 | 0.02 | 0.02 |
| **Example 33** | 0.06 | 0.06 | 0.02 | 0.02 | 0.04 |
| **Example 34** | 0.04 | 0.06 | 0.02 | 0.02 | 0.02 |
| **Example 35** | 0.08 | >0.1 | 0.02 | 0.02 | 0.04 |
| **Example 36** | 0.04 | (0.1) | 0.02 | 0.02 | 0.02 |
| **Example 37** | 0.08 | 0.1 | 0.02 | 0.04 | 0.04 |
| **Example 38** | 0.04 | 0.08 | 0.02 | 0.02 | 0.02 |
| **Example 39** | 0.08 | 0.08 | 0.02 | 0.04 | 0.04 |
| **Example 40** | 0.04 | - | - | 0.02 | 0.02 |
| **Example 41** | 0.08 | - | - | 0.04 | 0.04 |
| **Example 42** | 0.04 | - | - | 0.02 | 0.02 |
| **Example 43** | 0.08 | - | - | 0.04 | 0.04 |
| **Example 44** | 0.04 | - | - | 0.02 | 0.02 |
| **Example 45** | 0.08 | - | - | 0.04 | 0.04 |
| **Example 46** | 0.04 | - | - | 0.02 | 0.02 |
| **Example 47** | 0.08 | - | - | 0.04 | 0.04 |
| **Example 48** | 0.04 | - | - | 0.02 | 0.02 |

### Examples 49-50 Feed test

A Salmonella infantis inoculum was mixed evenly in an amount of 1500-3000 bacteria/g of feed, was mixed evenly into a commercial chicken feed preparation of which approximately 2/3 was grain, the rest primarily fish meal and soy. The antimicrobial composition tested was distributed into the contaminated feed as evenly as possible in an amount corresponding to a dose of 8 kg/metric ton. Peptone water was dosed into a control flask. The cultures were stored at room temperature in 1-liter Erlenmeyer flasks closed with aluminum foil. On each sample-taking day, 2 parallel samples of 10 g were taken from the cultures, and after Ultra-Turrax stirring, dilution series were made of the samples.

From the dilution tubes, sample was spread onto 50 µl agar plates on Petri dishes. After 24 h of incubation at 37°C, the formed bacterial colonies were counted. Example compositions 49-50 were according to the invention.

| **Example** | **Medium** | **Time during which Salmonella disappears** (2 parallel determinations) |
|---|---|---|
| Reference | peptone water | >30 d |
| Reference | solution 1 *) | 7.7 d |
| Example 49 | 95 % solution 1 | 5.5 d |
| | 5 % cetylpyridinium Cl⁻ | |
| Example 50 | 95 % solution 1 | 5.1 d |
| | 2.5 % Kloramin-T | |
| | 2.5 % cetylpyridinium Cl⁻ | |

| | | |
|---|---|---|
| *) 80 % formic acid, 2 % phosphoric acid and 18 % water | | |

### Example 51 Ensiling test

A preservation agent composition according to the invention was prepared by mixing 71.5 % formic acid, 18 % propionic acid, 4.5% benzoic acid, 4.0% octylamine and 2 % water, and by heating the mixture until the benzoic acid had dissolved. The reference composition contained approximately 56 % formic acid. 29 % propionic acid, 9 % benzoic acid, 5.5 % water, and 0.5 % monoglyceride to inhibit crystallization of the benzoic acid. The preservatives were compared in ensiling. The preservatives were sprayed into the grass in an amount which corresponded to a rate of 5 liters per one metric ton of feed. The silages were packed into plastic bags, which were closed tightly. In the zero test, no preservatives were used. The following results were obtained:

| | 0-test | Reference | Invention (Example 51) |
|---|---|---|---|
| pH, 1 wk | 4.1 | 4.3 | 4.1 |
| 1 mo | 3.9 | 4.0 | 4.2 |
| Lactic acid, g/kg dry | | | |
| 1 wk | 46 | 28 | 38 |
| 2 mo | 84 | 54 | 42 |
| Acetic acid, g/kg dry | | | |
| 1 wk | 18 | 8 | 4 |
| 2 mo | 22 | 15 | 7 |
| Sugars, g/kg dry | | | |
| 1 wk | 19 | 140 | 126 |
| 2 mo | 20 | 97 | 120 |
| Ammonia*), % of soluble nutrient protein | | | |
| 1 wk | 5.4 | 5.0 | 5.0 |
| 2 mo | 15.6 | 6.0 | 5.0 |
| Yeasts, cfu/g | | | |
| 1 wk | 300000 | <100 | <100 |
| 1 mo | 4400000 | 490000 | 3200 |
| Molds, cfu/g | | | |
| 1 wk | 330000 | <100 | <100 |
| 1 mo | 5100000 | 190000 | <100 |
| Lactobacilli, cfu/g | | | |
| 1 wk | 33000000 | 920000 | 210000 |
| 1 mo | 3800000000 | 120000000 | 20000 |

| | | | |
|---|---|---|---|
| *) contains also amines, calculated as ammonia. | | | |

The results show that in this test performed in laboratory conditions the lactic acid fermentation of the 0-test was very successful. However, the silage contains considerable amounts of molds and yeasts. When the reference agent was used, these amounts were clearly reduced, acetic acid fermentation decreased, and the sugar content of the silage was high. By the use of the composition according to the invention, molds and yeasts were best controlled, even though the concentration of benzoic acid, effective against them, was in the composition one-half of the respective concentration in the reference sample. Although the dosage proved to be too high for lactic acid bacteria, the analyses of the silage nevertheless yielded excellent results.

### Examples 52-55 Bag tests

Tests according to the preceding example were performed on ensiling in plastic bags by using the following compositions according to the invention:

| **Example** | **Composition** |
|---|---|
| 52 | 99.0 % solution 2 + 1.0 % OA |
| 53 | 99.0 % solution 2 + 1.0 % CPy |
| 54 | 99.0 % solution 2 + 1.0% BA + OA (molar ratio 1:1) |
| 55 | 99.0 % solution 2 + 1.0 % BA + CPy (molar ratio 1:1) |

- Solution 2:: 47 % formic acid, 23 % ammonium formiate, 30 % water
- BA =: benzoic acid
- OA =: octylamine
- CPy: cetylpyridine chloride

The analysis results after the test were:

| **Example** | ***1** | **pH** | ***2** | ***3** | ***4** | ***5** | ***6** |
|---|---|---|---|---|---|---|---|
| Example 52 | 19.0 | 4.3 | 11.2 | 1.5 | 5.5 | 3.4 | 1.8 |
| Example 53 | 20.0 | 4.0 | 13.9 | 0.5 | 7.3 | 5.2 | 1.1 |
| Example 54 | 19.5 | 4.0 | 13.4 | 0.2 | 7.5 | 4.7 | 0.7 |
| Example 55 | 20.2 | 4.1 | 13.6 | 0.7 | 7.0 | 6.9 | 0.7 |

| | | | | | | | |
|---|---|---|---|---|---|---|---|
| ^{*1}: dry matter content, % | | | | | | | |
| ^{*2}: lactic acid, g/100 g of dry silage | | | | | | | |
| ^{*3}: acetic acid, g/100 g of dry silage | | | | | | | |
| ^{*4}: free acids, g/100 g of dry silage | | | | | | | |
| ^{*5}: sugars, g/100 g of dry silage | | | | | | | |
| ^{*6}: ammonia and amines (calculated as ammonia), g/100 g of dry silage | | | | | | | |

Excellent ensiling results were obtained with all of the compositions.

## Claims

1. An antimicrobial composition, **characterized in that** it comprises an antimicrobial component having as subcomponents
a) a substituted or unsubstituted C₁-C₄-monocarboxylic acid in an amount of 50-99.9 % by weight,
b) a microbicidal or microbiostatic cationic organic nitrogen compound in an amount of 0.1-10 % by weight, and
c) an organic acid, preferably carboxylic acid, containing a C₅-C₂₂-alkyl or aryl group, in an amount of 0.1-10, preferably 0.5-5% by weight, the sum of subcomponents in said antimicrobial component adding up to 100% by weight, and subcomponents b) and c) forming an anionic-cationic complex and subcomponent b) being present in a molar ratio to subcomponent c) capable of forming an oily or waxy residue in case of separation from subcomponent a).

2. A composition according to claim 1, **characterized in that** the molar ratio of subcomponents b) and c) is at least 0.21.

3. A composition according to Claim 1 or 2, **characterized in that** the substituted or unsubstituted C₁-C₄-monocarboxylic acid is formic acid, acetic acid, propionic acid, lactic acid or any mixture thereof, most preferably formic acid or a mixture of formic acid and propionic acid.

4. A composition according to any one of the above claims, **characterized in that** the cationic nitrogen compound is an ammonia compound of the type R¹-N⁺(R²,R³,R⁴), where: R¹ is a C₂-C₂₂-alkyl or hydroxyalkyl chain; R² is a C₂-C₂₂-alkyl or hydroxyalkyl chain, the same as or different from R¹, or it is a benzyl or C₁-C₄-alkylbenzyl group, a C₁-C₄-alkyl or hydroxyalkyl group or a hydrogen atom; R³ and R⁴ are C₁-C₄-alkyl or hydroxyalkyl groups or hydrogen atoms.

5. A composition according to any one of claims 1-3, **characterized in that** the cationic nitrogen compound comprises a nitrogen-containing ring, such as a pyridyl ring, wherein the nitrogen has a positive charge.

6. A composition according to Claim 4 or 5, **characterized in that** the nitrogen compound comprises a cation which is cetylpyridinium, myristyl- or cetyltrimethylammonium, dodecyl-or myristylbenzyldimethylammonium, dioctyl-, didecyl- or didodecyldimethylammonium, or any combination thereof.

7. A composition according to any one of the above claims, **characterized in that** the organic acid forming subcomponent c) is one or several acids of the group benzoic acid, parahydroxybenzoic acid, salicylic acid, sorbic acid, caprylic acid, capric acid, lauric acid, myristic acid, myristoleic acid, palmitic acid, palmitoleic acid, linolic acid, linoleic acid or arachidonic acid.

8. A composition according to any of the above claims, **characterized in that** it comprises an antimicrobial component containing
a) a substituted or unsubstituted C₁-C₄-monocarboxylic acid in an amount of 50-99.9% by weight,
b) a microbicidal cationic organic nitrogen compound in an amount of 0.1-10 % by weight,
c) an organic acid containing a C₅-C₂₂-alkyl or aryl group, in an amount of 0.1-10 % by weight, and
d) water in an amount of 0-30 % by weight, the sum of subcomponents in said antimicrobial component adding up to 100% by weight.

9. A composition according to any of the above claims, **characterized in that** it additionally comprises a solid particulate carrier having an average particle size of 0.01-20 mm, and that the weight ratio of the antimicrobial component to the particulate carrier is within a range of 1:10 to 10:1, preferably within a range of 3:7 to 7:3.

10. A method for the preparation of a composition according to any one of the above claims, **characterized in that** subcomponent b) and subcomponent c) are first reacted with each other so that an anion-cation addition compound is formed between the cation of subcomponent b) and the anion of subcomponent c), which compound is subsequently mixed with subcomponent a).

11. A method according to Claim 10, **characterized in that** the anion-cation addition compound is alkylpyridiniumbenzoate, -parahydroxybenzoate or -salicylate; alkylbenzyldimethylammoniumbenzoate, -parahydroxybenzoate or -salicylate; alkyltrimethylammoniumbenzoate, -parahydroxybenzoate or -salicylate; or primary alkylammoniumbenzoate, -parahydroxybenzoate or -salicylate.

12. The **use** of a composition according to any of Claims 1-9 as an agent which kills harmful microbes or inhibits their growth in animal-feed raw materials and preparations.

13. The **use** according to Claim 12 for the preservation of silage or grain.

14. The **use** of a composition according to any of Claims 1-9 for the disinfection or improving the hygiene of packaging materials, apparatus and equipment, vehicles and other technical means and materials, footwear and other protective equipment, as well as animal sheds, silos and other premises.

## Patentansprüche

1. Antimikrobielle Zusammensetzung, **dadurch gekennzeichnet, daß** sie eine antimikrobielle Komponente umfaßt, die als Teilkomponente
a) 50-99,9 Gew.-% gegebenenfalls substituierte C₁-C₄-Monocarbonsäure,
b) 0,1-10 Gew.-% einer mikrobiziden oder mikrobistatischen kationischen organischen Stickstoffverbindung, sowie
c) eine organische Säure, vorzugsweise eine Carbonsäure, die eine C₅-C₂₂-Alkyl- oder -Arylgruppe enthält, in einer Menge von 0,1-10, vorzugsweise 0,5-5 Gew.-%, wobei die Summe der Teilkomponenten in der antimikrobiellen Komponente 100 Gew.-% beträgt und die Teilkomponenten b) und c) einen anionisch-kationischen Komplex bilden und die Teilkomponente b) im Vergleich zur Teilkomponente c) in solch einem Molverhältnis vorliegt, daß bei Trennung von der Teilkomponente a) ein öliger oder wachsartiger Rückstand gebildet werden kann, enthält.

2. Zusammensetzung nach Anspruch 1, **dadurch gekennzeichnet, daß** das Molverhältnis der Teilkomponenten b) und c) mindestens 0,21 beträgt.

3. Zusammensetzung nach Anspruch 1 oder 2, **dadurch gekennzeichnet, daß** es sich bei der gegebenenfalls substituierten C₁-C₄-Monocarbonsäure um Ameisensäure, Essigsäure, Propionsäure, Milchsäure oder eine beliebige Mischung davon, am stärksten bevorzugt um Ameisensäure oder eine Mischung von Ameisensäure und Propionsäure, handelt.

4. Zusammensetzung nach einem der vorhergehenden Ansprüche, **dadurch gekennzeichnet, daß** es sich bei der kationischen Stickstoffverbindung um eine Ammoniumverbindung des Typs R¹-N⁺(R²,R³,R⁴) handelt, wobei R¹ eine C₂-C₂₂-Alkyl- oder - Hydroxyalkylkette bedeutet, R² eine C₂-C₂₂-Alkyl- oder -Hydroxyalkylkette bedeutet, die gleich wie oder unterschiedlich zu R¹ ist, oder eine Benzylgruppe oder eine C₁-C₄-Alkylbenzylgruppe, eine C₁-C₄-Alkyl- oder - Hydroxyalkylgruppe oder ein Wasserstoffatom, R³ und R⁴ C₁-C₄-Alkyl- oder -Hydroxyalkylgruppen oder Wasserstoffatome bedeuten.

5. Zusammensetzung nach einem der Ansprüche 1-3, **dadurch gekennzeichnet, daß** die kationische Stickstoffverbindung einen stickstoffhaltigen Ring, wie einen Pyridylring, in dem der Stickstoff eine positive Ladung trägt, umfaßt.

6. Zusammensetzung nach Anspruch 4 oder 5, **dadurch gekennzeichnet, daß** die Stickstoffverbindung ein Kation umfaßt, bei dem es sich um Cetylpyridinium, Myristyl- oder Cetyltrimethylammonium, Dodecyl- oder Myristylbenzyldimethylammonium, Dioctyl-, Didecyl- oder Didodecyldimethylammonium oder um eine beliebige Kombination davon handelt.

7. Zusammensetzung nach einem der vorhergehenden Ansprüche, **dadurch gekennzeichnet, daß** es sich bei der die Teilkomponente c) bildenden organischen Säure um eine oder mehrere Säuren aus der Gruppe Benzoesäure, Parahydroxybenzoesäure, Salicylsäure, Sorbinsäure, Caprylsäure, Caprinsäure, Laurinsäure, Myristinsäure, Myristoleinsäure, Palmitinsäure, Palmitoleinsäure, Linolsäure oder Arachidonsäure handelt.

8. Zusammensetzung nach einem der vorhergehenden Ansprüche, **dadurch gekennzeichnet, daß** sie eine antimikrobielle Komponente, die folgendes enthält:
a) 50-99,9 Gew.-% einer gegebenenfalls substituierten C₁-C₄-Monocarbonsäure,
b) 0,1-10 Gew.-% einer mikrobiziden kationischen organischen Stickstoffverbindung,
c) 0,1-10 Gew.-% einer organischen Säure mit einer C₅-C₂₂-Alkyl- oder -Arylgruppe, sowie
d) ungefähr 0-30 Gew.-% Wasser, wobei die Summe der Teilkomponenten in der antimikrobiellen Komponente 100 Gew.-% beträgt, umfaßt.

9. Zusammensetzung nach einem der vorhergehenden Ansprüche, **dadurch gekennzeichnet, daß** sie zusätzlich einen festen teilchenförmigen Träger mit einer durchschnittlichen Teilchengröße von 0,01-20 mm umfaßt und daß sich das Gewichtsverhältnis der antimikrobiellen Komponente zu dem teilchenförmigen Träger in einem Bereich von 1:10 bis 10:1, vorzugsweise innerhalb eines Bereichs von 3:7 bis 7:3, bewegt.

10. Verfahren zur Herstellung einer Zusammensetzung nach einem der vorhergehenden Ansprüche, **dadurch gekennzeichnet, daß** die Teilkomponente b) und die Teilkomponente c) zuerst miteinander so umgesetzt werden, daß zwischen dem Kation der Teilkomponente b) und dem Anion der Teilkomponente c) eine Anionen-Kationen-Additionsverbindung gebildet wird, die anschließend mit der Teilkomponente a) vermischt wird.

11. Verfahren nach Anspruch 10, **dadurch gekennzeichnet, daß** es sich bei der Anionen-Kationen-Additionsverbindung um Alkylpyridiniumbenzoat, -parahydroxybenzoat oder - salicylat, Alkylbenzyldimethylammoniumbenzoat, - parahydroxybenzoat oder -salicylat, Alkyltrimethylammoniumbenzoat, -parahydroxybenzoat oder -salicylat, oder primäres Alkylammoniumbenzoat, -parahydroxybenzoat oder -salicylat handelt.

12. Verwendung einer Zusammensetzung nach einem der Ansprüche 1-9 als Mittel, das schädliche Mikroorganismen abtötet oder ihr Wachstum in Tierfuttermittelrohstoffen und -produkten hemmt.

13. Verwendung nach Anspruch 12 zur Konservierung von Silage oder Getreide.

14. Verwendung einer Zusammensetzung nach einem der Ansprüche 1-9 zur Desinfektion oder Verbesserung der Hygiene von Verpackungsmaterialien, Apparaten und Geräten, Fahrzeugen oder anderen technischen Mitteln und Materialien, Fußbekleidung und anderer Schutzausrüstung, sowie Ställen, Silos und anderen Anlagen.

## Revendications

1. Composition antimicrobienne **caractérisée en ce qu'**elle comprend un composant antimicrobien présentant, comme sous-composants,
a) un acide C₁-C₄-monocarboxylique substitué ou non-substitué en une quantité de 50 à 99,9% en poids,
b) un composé azoté organique cationique microbicide ou microbiostatique en une quantité de 0,1 à 10% en poids, et
c) un acide organique, de préférence un acide carboxylique, possédant un groupe alkyle en C₅-C₂₂ ou aryle, en une quantité de 0,1 à 10, de préférence de 0,5 à 5% en poids, la somme des sous-composants dans ledit composant antimicrobien allant jusqu'à 100% en poids, et les sous-composants b) et c) formant un complexe anionique-cationique et le sous-composant b) étant présent dans un rapport molaire avec le sous-composant c) capable de former un résidu huileux ou cireux en cas de séparation du sous-composant a).

2. Composition selon la revendication 1, **caractérisée en ce que** le rapport molaire entre les sous-composants b) et c) est d'au moins 0,21.

3. Composition selon la revendication 1 ou 2, **caractérisée en ce que** l'acide C₁-C₄-monocarboxylique substitué ou non substitué est l'acide formique, l'acide acétique, l'acide propionique, l'acide lactique ou des mélanges quelconques de ceux-ci, très préférentiellement l'acide formique ou un mélange d'acide formique et d'acide propionique.

4. Composition selon l'une quelconque des revendications ci-dessus, **caractérisée en ce que** le composé azoté cationique est un composé ammoniacal du type R¹-N⁺(R²,R³,R⁴), où : R¹ est une chaîne hydroxyalkyle ou alkyle en C₂-C₂₂ ; R² est une chaîne hydroxyalkyle ou alkyle en C₂-C₂₂, identique ou différente de R¹, ou est un groupe benzyle ou C₁-C₄-alkylbenzyle, un groupe hydroxyalkyle ou alkyle en C₁-C₄ ou un atome d'hydrogène ; R³ et R⁴ sont des groupes hydroxyalkyle ou alkyle en C₁-C₄ ou des atomes d'hydrogène.

5. Composition selon l'une quelconque des revendications 1 à 3, **caractérisée en ce que** le composé azoté cationique contient un noyau azoté, tel qu'un noyau pyridyle, dans lequel l'azote possède une charge positive.

6. Composition selon la revendication 4 ou 5, **caractérisée en ce que** le composé azoté contient un cation qui est un cétylpyridinium, un myristyl- ou cétyltriméthylammonium, un dodécyl- ou myristylbenzyldiméthylammonium, un dioctyl-, didécyl- ou didodécyldiméthylammonium, ou une combinaison quelconque de ceux-ci.

7. Composition selon l'une quelconque des revendications ci-dessus, **caractérisée en ce que** l'acide organique formant le sous-composant c) est un ou plusieurs acides parmi le groupe constitué de l'acide benzoïque, de l'acide para-hydroxybenzoïque, de l'acide salicylique, de l'acide sorbique, de l'acide caprylique, de l'acide caprique, de l'acide laurique, de l'acide mysristique, de l'acide myristoléique, de l'acide palmitique, de l'acide palmitoléique, de l'acide linolique, de l'acide linoléique ou de l'acide arachidonique.

8. Composition selon l'une quelconque des revendications ci-dessus, **caractérisée en ce qu'**elle comprend un composant antimicrobien comprenant
a) un acide C₁-C₄ monocarboxylique substitué ou non-substitué en une quantité de 50 à 99,9% en poids,
b) un composé azoté organique cationique microbicide en une quantité de 0,1 à 10% en poids,
c) un acide organique contenant un groupe alkyle en C₅-C₂₂ ou aryle en une quantité de 0,1 à 10% en poids, et
d) de l'eau en une quantité de 0 à 30% en poids, la somme des sous-composants dans ledit composant antimicrobien allant jusqu'à 100% en poids.

9. Composition selon l'une quelconque des revendications ci-dessus, **caractérisée en ce qu'**elle comprend en outre un support particulaire solide présentant une taille de particule moyenne de 0,01 à 20 mm, et que le rapport pondéral entre le composant antimicrobien et le support particulaire est compris dans un intervalle de 1:10 à 10:1, de préférence dans un intervalle de 3:7 à 7:3.

10. Méthode de préparation d'une composition selon l'une quelconque des revendications ci-dessus, **caractérisée en ce que** le sous-composant b) et le sous-composant c) réagissent tout d'abord l'un avec l'autre de telle sorte qu'il se forme un composé d'addition anion-cation entre le cation du sous-composant b) et l'anion du sous-composant c), lequel composé est ensuite mélangé avec le sous-composant a).

11. Méthode selon la revendication 10, **caractérisée en ce que** le composé d'addition anion-cation est un alkylpyridiniumbenzoate, -parahydroxybenzoate ou - salicylate ; un alkylbenzyldiméthylammoniumbenzoate, -parahydroxybenzoate ou -salicylate ; un alkyltriméthylammoniumbenzoate, -parahydroxybenzoate ou - salicylate ; ou un alkylammoniumbenzoate, -parahydroxybenzoate ou -salicylate primaire.

12. Utilisation d'une composition selon l'une quelconque des revendications 1 à 9 en tant qu'agent détruisant des microbes nuisibles ou inhibant leur croissance dans des matières premières et des préparations de nourriture pour animaux.

13. Utilisation selon la revendication 12 pour la conservation de fourrage ensilé ou de céréales.

14. Utilisation d'une composition selon l'une quelconque des revendications 1 à 9 pour la désinfection ou l'amélioration de l'hygiène de matériaux, d'appareils et d'équipement d'emballage, de véhicules et d'autres moyens techniques et matériaux, de chaussures et autres matériels protecteurs, ainsi que de logements animaliers, de silos et d'autres locaux.
